# EUROPEAN PATENT SPECIFICATION

(11) **EP 3 393 356 B1**
(45) Date of publication and mention of the grant of the patent: **08.05.2019**
(21) Application number: 16822932.6
(22) Date of filing: 15.12.2016
(51) Int. Cl.: A61B 6/00, A61B 8/08, A61B 8/00, G01N 29/28, G21K 1/02

(54) **ULTRASOUND COMPATIBLE X-RAY ANTI-SCATTER GRID**
ULTRASCHALLKOMPATIBLES RÖNTGENSTRAHLENSTREURASTER
GRILLE ANTI-DISPERSION À RAYONS X COMPATIBLE AUX ULTRASONS

(30) Priority: 21.12.2015 EP 15201523
(43) Date of publication of application: 31.10.2018
(73) Proprietor: Koninklijke Philips N.V., 5656 AE Eindhoven (NL)
(72) Inventor: SIMON, Matthias, 5656 AE Eindhoven (NL); RUETTEN, Walter, 5656 AE Eindhoven (NL); SOLF, Torsten, 5656 AE Eindhoven (NL)
(74) Representative: Ledeboer, Johannes Albertus
(86) International application number: PCT/EP2016/081089
(87) International publication number: WO 2017/108544

(56) References cited:
- EP-A2- 0 087 844
- US-A- 5 474 072

## Description

### FIELD OF THE INVENTION

The invention relates to an anti-scatter grid, a detector module and an imaging apparatus.

### BACKGROUND OF THE INVENTION

More and more imaging applications combine X-ray imaging with ultrasound (US) imaging. A mammography system with separate detectors for X-ray and ultrasound is reported in US 2008/0242979.

However, this type of multi-modal imaging has proved cumbersome in applications. For instance, some present setups require imaging in two subsequent procedures, one for X-ray the other for ultrasound (US). Also, the image quality in such settings remained behind expectations. Document US 5 474 072 A (SHMULEWITZ, A., 12 December 1995) may be considered to disclose an anti-scatter assembly according to the preamble of claim 1.

### SUMMARY OF THE INVENTION

There may therefore be a need for improved multimodal US and X-ray imaging.

The object of the present invention is solved by the subject matter of the independent claims where further embodiments are incorporated in the dependent claims. According to a first aspect of the invention there is provided an anti-scatter assembly for X-ray imaging including a grid filled with a filler material, said filler material having an acoustic impedance that corresponds to that of human or animal tissue.

The proposed anti-scatter grid (ASG) can be used in combined X-ray and ultrasound imaging for concurrent imaging. The overall US and X-ray imaging can be done in a single procedure rather than subsequently in two imaging procedures. The filled ASG can be integrated into a single detector module alongside an X-ray detector and an ultrasound transducer. The ASG can remain in place even during US imaging.

Earlier ASGs were intransparent to US and this may entice users of combined US and X-ray imagers to dispense with ASG altogether which in turn caused the X-ray image quality to remain under par. The proposed ASG on the other hand encourages use in combined X-ray and US imaging due to its US transparency thus ensuring high-quality X-ray and ultrasound imaging at the same time.

According to one embodiment, the acoustic impedance is in the range of 1.4 MRayls to 1.8 MRayls. Specifically, according to one embodiment, the acoustic impedance is around 1.5 MRayls. These specific acoustic impedances have been found to provide a good approximation of specific acoustic impedance for range of human or animal tissues. Structurally, and according to one embodiment, the filler material is a gel. According to one embodiment, the filler material includes silicone. According to one embodiment, the filler material includes a polymer.

According to one embodiment, the distance between lamellae of the grid matches half a wavelength of an ultrasound center frequency. This allows achieving yet better US transparency.

According to one embodiment, the grid has a 2D structure, in particular a honeycomb structure, formed from cells. A cell-center-to-cell-center pitch matches half the wavelength of the ultrasound center frequency.

According to one embodiment, a pitch of the grid is smaller than a tenth of a wavelength of an ultrasound center frequency.

Medical ultrasound transducers contain more than one operating frequency. The following frequencies are a guide to center frequencies typically used for ultrasound examination:
2.5 MHz: deep abdomen, obstetric and gynaecological imaging;
3.5 MHz: general abdomen, obstetric and gynaecological imaging;
5.0 MHz: vascular, breast, pelvic imaging;
7.5 MHz: breast, thyroid;
10.0 MHz: breast, thyroid, superficial veins, superficial masses, musculoskeletal imaging;
15.0 MHz: superficial structures, musculoskeletal imaging;
20.0 - 45.0 MHz: intravascular ultrasound.

According to one embodiment, the assembly includes a retainer component so as to retain the filler material within the grid.

According to one embodiment, the retainer component includes at least one cover portion coupled to a radiation egress or radiation ingress face of said grid.

According to one embodiment, the assembly includes a coupling structure (such as a soft silicone US matching layer) to promote good ultrasound coupling into the body of a patient. This structure can be provided onto the grid (on the radiation ingress side) which is to contact the body. Alternatively, the coupling structure forms only temporary part of the assembly. For instance, the coupling structure may be inserted into a patient support structure. The assembly is then coupled to the patient when contact is made by the assembly via the coupling structure.

According to one embodiment, the coupling structure is a disposable and is provided by a manual or automatic dispenser system. The dispenser system may be attached to the assembly or to the patient support structure such as a patient table. According to one embodiment, the grid comprises at least two lamellae with an interspace in between and wherein said interspace is completely filled with said filler material.

In particular, the grid is filled at the exclusion of air enclosures.

According to a further aspect, there is provided a detector module, comprising: an ultrasound transducer; and
an assembly as per any one of the previous claims coupled to said ultrasound transducer.

According to one embodiment, the module further comprises an X-ray detector.

According to one embodiment, the ultrasound transducer, the anti-scatter assembly and the X-ray detector are arranged in-line. In particular, ultrasound waves pass through the anti-scatter assembly to reach the ultrasound transducer. This allows a compact arrangement and convenient multi-modal imaging operation.

According to one embodiment, the ASG assembly is arranged in front of said ultrasound transducer relative to an ultrasound propagation direction.

According to one embodiment, the X-ray detector and the ultrasound transducer are arranged on opposing faces of one and the same wafer. "Wafer" includes any one of "silicon wafer", "silicon detector plane" or "combined solid state imaging plane", or other.

According to one embodiment, the X-ray detector and the ultrasound transducer are arranged on respective wafers, and the two wafers are coupled back-to-back. According to a further aspect, there is provide a combined X-ray and Ultrasound imaging system comprising a detector module as per any one of the above mentioned embodiments or comprising an assembly as per any one of the above mentioned embodiments.

According to a further aspect, there is provided an imaging system, comprising:
an X-ray imaging component;
an US-imaging component, and
an anti-scatter device for said X-ray imaging component.

In one embodiment, the anti-scatter grid device is arrangeable, during operation of the US imaging component, in between an X-ray source of the X-ray component and an ultrasound detector of said US imaging component. This allows easy operation of the imaging system as the anti-scatter device does not have to be removed before one uses the ultrasound imaging component.

A method of manufacturing of an anti-scatter device assembly, comprising: providing a grid structure with at least two lamellae having an interspace defined in between; and
arranging a filler material into said interspace. This can be achieved by filling or forcing the filler material into said interspace.

Alternatively, the anti-scatter device is built-up by depositing, in alternation, strips of lamella material and of filler material either on top of each other or side by side.

The term "anti-scatter assembly (device)" as used herein includes anti-scatter grid (ASG) devices and collimators, in particular post-collimators and other devices that have a grid structure and are suitable to reduce X-ray scatter.

### BRIEF DESCRIPTION OF THE DRAWINGS

Exemplary embodiments of the invention will be described with reference to the following drawings wherein:
Figure 1 shows a multi-modal imaging system
Figure 2 shows different embodiments of an entire scatter grid assembly; and
Figure 3 shows a cross section of different embodiments of a hybrid US/X-ray detector module.

### DETAILED DESCRIPTION OF EMBODIMENTS

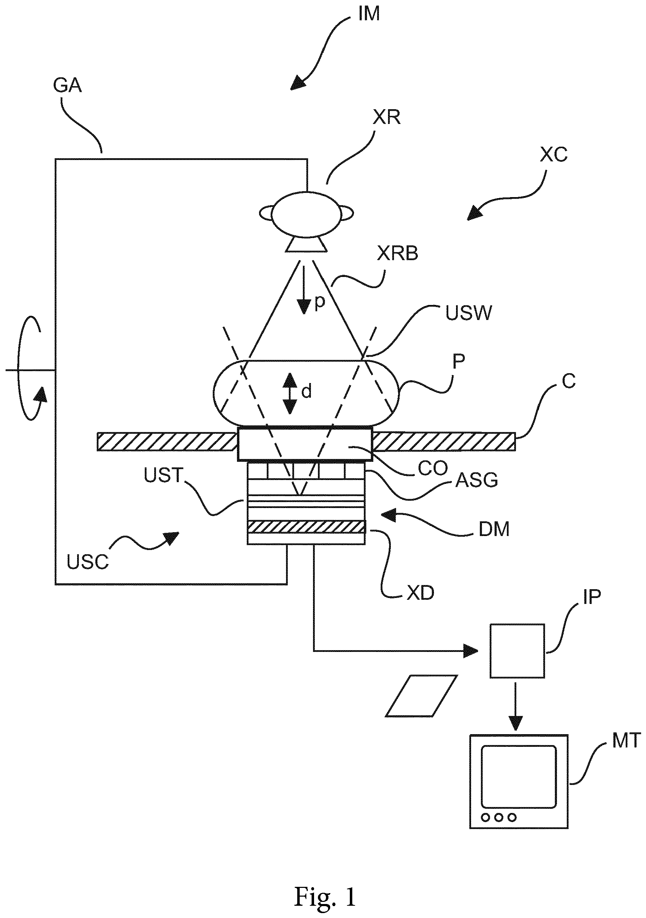

With reference to Figure 1, there are shown components of an imaging system IM.

The imaging system IM is multimodal in the sense that it combines two imaging components that work on different principles: there is an X-ray imaging component XC and there is an ultrasound imaging component USC. Possible, non-exhaustive areas of application include mammography or cardio-vascular imaging.

Each imaging component can be made to work separately and independent from each other. One component may be used to acquire a first image set and, subsequently thereto, the other component may be used to acquire a second image set. Preferably both components, ultrasound and X-ray, can work concurrently to acquire image sets at the same time of an object to be imaged P. The object is a human or animal (in particular mammal) patient. What is mainly envisaged herein is a human patient or an anatomical part thereof. In the following we will therefore refer to the object as patient P. In one embodiment, patient P is supported on a support structure C such (as a couch or table or other) during the imaging.

Both imaging modalities, ultrasound US and X-ray, allow querying for information about the internal structure of the patient P. The ultrasound imagery and the X-ray imagery can then be viewed separately from each other. Preferably, the image streams from the two modalities can be fused into a combined image stream by suitable registration techniques implemented by an image processing component IP. This component includes a visualizer that maps the image values to suitable color or grey values. These values are then used to drive suitable video circuitry to effect display on a display unit MT of the US imagery or of the X-ray imagery or of the combined image. For instance, in the combined image, the two image sets may be overlaid onto each other (possibly rendered with different color-coding) thereby complementing each other visually and in image content. More specifically, image structures (such as soft-tissue) not ordinarily discernable in an X-ray, can still be shown by overlaying, on the X-ray image, soft tissue image structures from the US imagery. Another visualization option includes overlaying on the X-ray image functional information, such as flow or velocity information obtained from the US component. This combined X-ray+US visualization includes preferably geometric corrections as the US information is usually distorted.

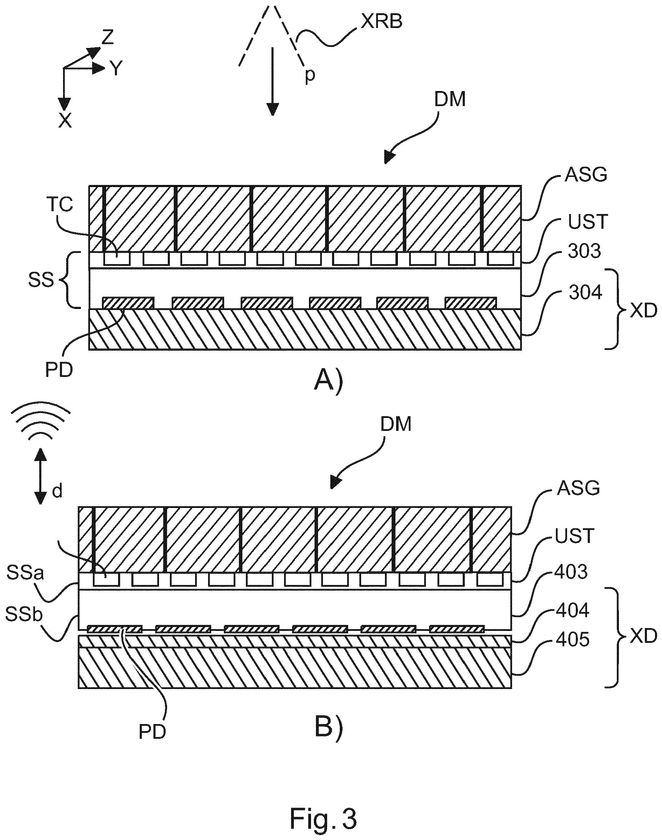

Turning first to the ultrasound component USC, this comprises an ultrasound transducer UST. Preferably, but not necessarily the ultrasound transducer UCT is of the CMUT type (capacitive micro-machined ultrasound transducer). The underlying contrast principle in ultrasound imaging is the varying degrees at which ultrasound is reflected off different tissue structures. The ultrasound transducer UST operates two-fold: it generates ultrasound waves USW that propagate along direction *d* (opposite X-ray direction *p*) towards the patient. The outgoing waves are then reflected off tissue types at different degrees of intensity. The reflected, incoming, waves then travel back to the transducer UST and are then detected there. Generation and detection of ultrasound is via a plurality of transducer cells TC (see Fig 3). The transducer cells are arranged linearly in a 1D array or, preferably, are arranged in row and columns in a 2D array. The arrangement in either 1D or 2D follows a certain "pitch", that is, a certain spatial frequency (# of transducer cells/unit length). In CMUT, each transducer cells TC is organized around a tiny cavity or depression formed in a silicon substrate SS. There is a respective membrane portion covering each cavity to form a plurality of cavity-membrane structures, one for each cell TC. A pair of respective electrodes are arranged to sandwich each cavity-membrane structure to form the respective cell TC. The outgoing ultrasound wave USW is generated by urging the membrane to vibrate upon application of a voltage across the pair of electrodes. Conversely, the membrane can be made to vibrate by the incoming (reflected) sound waves and this information is then converted into voltage fluctuations signals. The voltage signals are then converted by A/D circuitry into digital signals which can be compiled by the image processor IP into an ultrasound (US) image. The US image may be stored or processed such as rendered for view on the display unit. The US frequency used in medical US imaging is typically in the range of 1.5 - 3.5 Mhz but variations from this range may be contemplated in certain (non-medical) contexts.

Turning now in more detail to the X-ray component XC, this comprises an X-ray source XR such as an X-ray tube and an X-ray radiation sensitive detector XD assembly. The detector assembly may be a flat panel detector unit (FPD) either of the indirect conversion type or of the direct conversion type.

Detector assemblies XD of the indirect conversion type comprise a scintillation layer formed, for instance, from Cesium Iodide or Gadolinium Oxysulfide or any other suitable material. The scintillation layer operates to convert impinging X-ray radiation into light which is detected by a system of photo-diodes arranged opposite the scintillation layer. The photo-detectors translate the detected light into electrical signals. The system of photodiodes is arranged as detector pixels in a 2D array in rows and columns. However, a mere 1D structure of detector pixels as used in fan beam CT is not excluded herein.

Detector assemblies XD of the direct conversion type can dispense with the scintillation layer and are formed from a material (eg, amorphous Selenium) that can directly translate impinging X-ray radiation (via electron-hole pairs that form in the layer) into electrical charge signals via a system of electrodes. The electrodes are arranged in rows and or columns to define the detector pixels.

Independent of the X-ray detector type and similar to the system of transducer cells explained above, the respective system of detector pixels is laid out at a suitable pitch (spatial frequency of the detector pixels). Furthermore, the detector XD includes suitable read-out circuitry to collect the various electrical signals. Suitable A/D circuity then converts the electrical signals into digital signals which can then be processed by the image processing module IP into a standalone or fused image. Just like the US imagery, the X-ray imagery can be stored, processed or displayed in isolation or together with the US imagery on the display device MT.

The underlying image contrast conferring principle in X-ray is the variation of density within the object. The X-ray radiation is provided in the form of an X-ray beam XRB that propagates along a main direction *p* from the X-ray source through the patient and then towards the X-ray detector XD. The X-ray radiation as emitted from the X-ray source experiences different degrees of attenuation as a function of tissue density. The varying degrees of attenuation are encoded in the radiation that egresses the patient at a distal side relative to the X-ray source, which is then detected by the detector XD. Unfortunately, the information content encoded in the detected radiation is usually compromised by scatter contributions incurred whilst the X-radiation passes through the object. Attenuation of the X-radiation in interaction with matter in patient P is down to absorption and scatter. It is mainly the absorption component, which one wishes to image. The scatter contribution, consisting of Rayleigh and Compton scattering, is often considered a nuisance because it decreases image contrast. To reduce this scatter contribution (and thus increasing image contrast), the imaging system IM includes an anti-scatter grid ASG that includes a grid structure defined by a system of lamella formed from lead, tungsten or other high Z-material to block out scatter.

During an image acquisition procedure, the X-ray radiation and the sound waves are generated and interact with the patient. The patient resides (possibly on the support structure C) in between the X-ray source and a detector module DM. In one embodiment it is envisaged to integrate not only the X-ray detector XD and the ultrasound transducer UST into said detector module DM, but also the ASG. Specifically, the three components, ASG, X-ray detector XD and ultrasound transducer UST may be arranged in-line relative to the X-ray source XR. Furthermore, the three components, ASG, X-ray detector XD and ultrasound transducer UST may be arranged in a single housing or frame. This integrated, in-line arrangement is possible, because, unlike ASG's used in conventional X-ray imaging, the ASG proposed herein has a structure that makes it not only transparent for ultrasound waves but also compatible with ultrasound imaging. More particularly, the proposed ASG, unlike conventional ASGs, is filled with a filler material that has an acoustic impedance that matches those of human or animal (in particular mammal) tissue. Because of this match-up, the ASG can remain within the ultrasound cone USW generated by the transducer USC during the US imaging. Specific acoustic impedance is a material property. It is a measure for the particle velocity induced by a given acoustic pressure in a given material. The filler material in the ASG as proposed herein serves as a US impedance adaptor component to gently adapt US wave impedance (from and into the transducer UST) to the expected in-tissue wave propagation speed, thus reducing, if not entirely eliminating, unwanted US reflections. This ensures good US image quality even though the ultrasound wave travels through the ASG assembly during US imaging. Conventional ASGs, because of the mismatching acoustic impedance of (in particular) their lamella, cause prohibitive reflection patterns which would compromise ultrasound image quality. To be sure, even in the proposed ASG, the acoustic impedance of the lamellae remains unchanged. However, in the proposed ASG, the lamellae are embedded within the filler material having the right acoustic impedance to match that of human or animal tissue. As a result, the average acoustic impedance taken across the footprint of the ASG better matches the in-tissue US impedance compared to conventional ASGs without such a filler material.

The combined detector module DM as envisaged herein in one embodiment has a layered structure, with the ASG arranged proximal to the X-ray source as the top layer. The ASG is coupled then to the ultrasound transducer UST a middle layer and the bottom layer is formed by the X-ray detector XD. Furthermore, the three components ASG, UST and XD are arranged in line during in particular US imaging. The ASG is proximal to the X-ray source on top, and the US ultrasound transducer UST and the X-ray detector XR arranged distal to the X-ray source behind the ASG. Of course, one may still remove the ASG from the US/X-ray field of view if desired, for instance if one wishes to use only the US imaging component US. In this case, the proposed imaging system affords to the user the flexibility to still leave the ASG within the field of view, no matter which imaging component (X-ray or US) is being used. This allows reducing complexity of the imaging apparatus and allows for compact build or used in conditions where space is a premium. It will be understood from the above that the spatial qualifiers" top" "proximal" and "bottom" "distal " "behind" or "front" as used herein are taken relative to the location of the X-ray source. In other words top or proximal means closer to the X-ray source whereas bottom or under or distal means further away from the X-ray source XR.

In combined use of the imager IM, that is, during X-ray and ultrasound imaging acquisition, the detector module DM is urged into contact with the patient's body. Coupling is achieved in one embodiment with a coupling structure CO having an appropriate acoustic impedance (close to 1.5Mrayls). The coupling structure CO allows creating a relatively large area of air free contact with the patient. Preferably, the coupling structure is a package/cushion of soft or liquid US contact gel for patient comfort. A layer of silicone may also be used instead. In one embodiment the coupling structure CO is a discrete structure permanently coupled to the radiation ingress side of the grid assembly ASG. Preferably however the coupling structure CO is a disposable, so is removably coupled to the ASG via suitable fastening arrangement such as Velcro or others. This allows hassle-free exchanging of the coupling structure CO for a new one because of wear or hygiene. The fastening arrangement is arranged outside the US path, for instance on the sides of the ASG. In one embodiment, the (disposable) coupling structure CO is provided by an automatic dispenser system attached to the ASG or to the patient support C. Preferably the support structure C is X-ray and US transparent throughout or only at a designated region at which contact of the patient with the detector module occurs.

For instance, the support structure C may include a suitably defined clearance or hole (as shown in Figure 1) through which the contact with the patient's body can be established via the coupling structure CO. The coupling structure CO may be fixedly arranged as an inset in said hole. Alternatively, no such clearance in the support structure C is required, if the gantry is arranged to allow the detector module to approach the patient from above. In this case the X-ray exposure source is positioned under the patient and support C.

During imaging, the patient is positioned between the detector module DM on one side and the X-ray source XR on the other. The X-ray source XR is then positioned at a suitable imaging distance from the patient. X-ray image acquisition can then commence together with the ultrasound image acquisition to acquire the two sets of image data, which can then be processed into images by the image processing system IP. In one embodiment, the X-ray source and the detector module are arranged on a common gantry GA. The gantry may be rotatable or otherwise moveable to suitably position the X-ray source XR and/or the detector module DM relative to the patient P to be able to acquire relevant imagery of a particular part of the human anatomy. However, the gantry GA is optional because a purely wall mounted or floor mounted imaging systems IM are also envisaged herein where the patient is actually standing in a room in a designated area with the X-ray source and the detector module each being suitably mounted within the room but not necessarily physically coupled to each other as shown in Fig 1 at the example of a C-arm type imager IM. It should be also understood that the proposed ASG can be used as an add-on to any existing combined US and X-ray imagers.

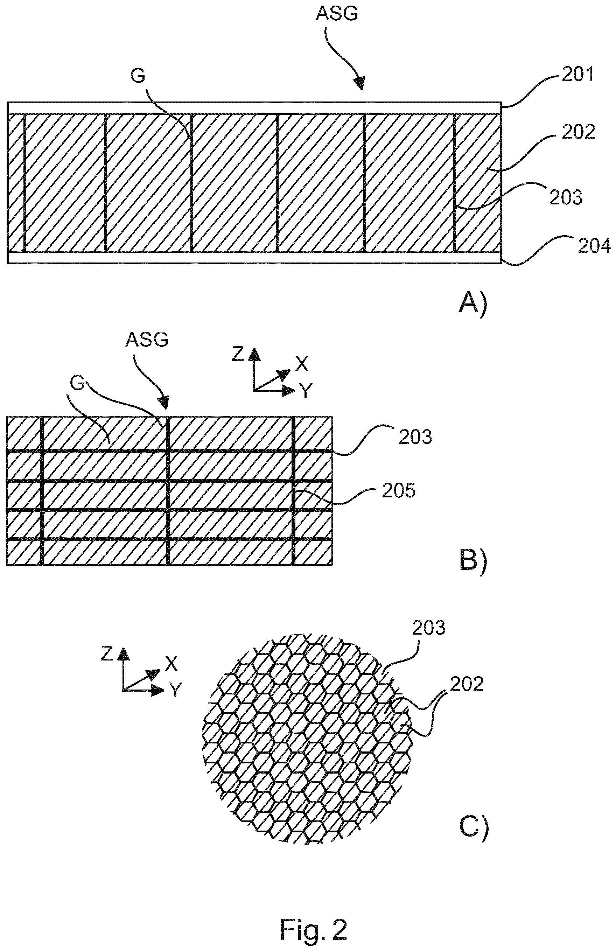

Turning now to Figure 2, this shows in more detail different views and embodiments of the proposed ASG.

Figure 2A shows a cross sectional side elevation of the ASG assembly as envisaged herein. The ASG assembly comprises the grid structure which is either 1D (one-dimensional) or 2D (two-dimensional). The grid is formed by a system of small walls or strips, the lamella 203 that is, formed from lead, tungsten or other high Z material to block off the unwanted scatter contribution within the X-ray radiation. A 2D ASG can provide higher mechanical stiffness than 1D. This is useful in particular when using relatively soft materials like lead for the lamellae. Lead as lamella material can be advantageous as it provides lower acoustic impedance (compared to tungsten) and this allows US wave propagation even at oblique angles.
In the 1D arrangement the lamella 203 are organized as a single set in parallel that runs along single direction (in Z direction according to Figure 2A) at a regular distance apart. In one embodiment of a grid with 2D structure, there are two sets of parallel lamellae 203 that run across each other to form a rectangular grid made up from cells bounded by the lamellae. However, the 2D structure may not necessarily be realized as a rectangular grid as shown in Figure 2C where the lamella are arranged as a honeycomb structure where the lamellae form non-rectangular cells such as round, polygonal (eg hexagonal) cells or cells of other shapes. The honey comb ASG acts like a waveguide for the ultrasound, due to the high aspect ratio of 5-10 and the US reflecting walls. However, this is no problem as the image resolution for the US is usually above 250µm. No matter whether the ASG is arranged as a 1D or a 2D structure, interspaces are formed between the lamellae. Some or all of these interspaces is filled as proposed herein with a filler material 202 that has an acoustic impedance that corresponds to that of human tissue as mentioned above.

The lamella for the grid can be by manufactured by stamping and staging of thin foils, not unlike approaches used in the manufacturing of similar collimators for SPECT. Preferably a given interspace between two lamellae is completely filled with the filler material 202. More particularly the filler material extends the full length, width and height of the interspace. More particularly there are no or minimal air enclosures in the inter-space once filled with filler material 202. Preferably the filler material is made from a polymer. For instance the filler material may be applied as a type of glue, in particular an acrylic glue. Instead of or in addition, a gel material may be used as filler material 102. In embodiment the filler material is a conventional US gel as currently used in conventional ultrasound imaging for application on the patient's skin before contact is made with the ultrasound probe. In another embodiment a silicone or silicone gel is used as filler material 202. Silicone is advantageous as it provides mechanical stiffness and relatively low ultrasound absorption. RTV (room temperature vulcanization) silicone is particularly advantageous in this respect as Silicone has an acoustic impedance close to water (there is only a 5% difference) thus is ideal to mimic a range of human or animal tissues and it has proven long term stability. In particular a (clear) silicone sealant may be used.

In any of the above filler material embodiments, the (specific) acoustic impedance of the material is preferably chosen to best match a range of human or animal tissues. Generally, the acoustic impedance Z is given by the speed of sound v in the material times its density p: Z=ν^{∗}ρ. As mentioned, in one embodiment the material has a specific acoustic impedance similar to water. Preferably, the acoustic impedance should be within 1.4-1.8 MRayls. Also, a range of 1.4-1.99 MRayls may be acceptable. Applicant has found that this is the relevant acoustic impedance interval for human tissue impedance and allows reducing reflections. Relevant human tissue (materials) include water, blood, far, liver tissue, brain tissue, kidney tissue, heart tissue, muscle tissue, eye tissue and skin. The more one departs outside this interval, the more the reflections will increase. Preferably and ideally, the closer the acoustic impedance is to 1.5MRayls the better in terms of US reflection reduction. In particular a (clear) silicone sealant may be used having an acoustic impedance of about 1.53 MRayls. Other suitable more specific exemplary embodiments include Ethyl-vinylacetate (about 18 % or 28% Acetat) at 1.6 or 1.69 MRayls or (low density) Polyethylene at 1.73-1.79 MRayls.

It should be noted that the acoustic impedance of the filler material is established under test conditions at a specific temperature etc. Also, acoustic impedance may be described in other quantities than the one shown above and all these are equivalent definitions of the acoustic impedance are envisaged herein.

In one embodiment, the ASG assembly further comprises one or more retainer components to ensure the filler material remains within the grid. In one embodiment, the retainer component comprises two cover layers 201, 204, one 201 arranged on top (on the radiation ingress face) of the grid and the other on the bottom (radiation egress face) of the grid. This construction does not only keep the filler material in place but also acts to stiffen the whole grid against bending. The retainer component material may be made less US transparent than the filler itself because the retainer component should have a higher rigidity. In one embodiment, the top and back covers 201, 204 include low density polyethylene(LDPE) to provide mechanical and stiffness and allow precise mechanical alignment with the ultrasound transducer UST and the X-ray detector XD.

Preferably, at least one or both covers 201, 204 have a thickness of multiples of the half central wavelength of the sound waves to be used. This allows reducing artifacts in the US imagery. Although two cover portions as shown in the figure are preferable, in an alternative embodiment only single cover 204 is used at the egress face of the grid.

As another alternative, rather than having two discrete retainers (top and bottom cover) other solutions are also envisaged herein. For instance, the whole of the grid may be suitably encapsulated such as shrinkwrapped in a plastic foil to ensure the filler material remains in place.

The top cover of the ASG can also be used as detector cover, directly interfacing the patient via the impedance matching coupling structure CO.
The working ability of the ASG is described by a ratio between i) the spatial frequency of the lamellae and ii) the depth or height of the lamellae (measured in propagation direction of the X-radiation). This ratio is sometime referred to as the grid or aspect ratio. For 2D grids, there are then two ratios, one for each direction. Grid ratios of 1:4, 1:8 or 1:12 are common but other values may also be called for in certain applications. The "grid pitch", that is, the inverse of the spatial frequency of the lamellae series, is in general equal or an integer multiple of the pitch of the X-ray detector XD pixels or slightly lower. For example, the spatial frequency may be in the range of 30-60 lamella/cm. If the pitch of the lamellae is around 250 µm, the height of the grid lamella is about 1-2 mm. In one embodiment, the grid pitch also corresponds to that of the transducer cell matrix in ultrasound transducer UST. Using an ASG whose pitch matches the detector pitch of the ultrasound array and the X-ray pixel pitch (or a multiple of the pitch) allows preventing or reducing Moiré effects. In X-ray detectors, the grids are usually not perfectly aligned to the pixel matrix, so some mismatch with the X-ray pixel pitch is acceptable to accommodate the pitch of the US transducer array. A lamellae distance of between 200-500µm has proved particularly advantageous as this fulfills most pitch requirements for both, US and X-ray.

The accuracy of the spatial arrangements of the lamella in accord with a required aspect ratio is important for their working. Also it is preferable to have the lamella run in parallel with satisfactory precision. To ensure this in particular for the case of 1D grids, a set of spacers 205 are used that run across the lamellae to afford the stability. This is shown in Figure 2B. More particularly Figure 2B shows a plan view in x direction (that is, in the direction of travel of the X-ray beam XB or parallel to the propagation direction of the ultrasound wave USW). 2D grids are inherently more stable and no such spacer elements are necessary. Indeed, the grid as shown in Fig 2B counts as a 1D grid, in particular, because the spacers 205 are not required to follow the aspect ratio or pitch of the lamella 203. In some embodiments, the lamellae are slanted for focus with a focal spot of the X-ray source. The slanting increases for lamella further away from the ASG center. This allows more efficient use of the radiation. To achieve yet better US compatibility of the grid, the ASG pitch is preferably smaller than a tenth of the ultrasound center frequency.

In one embodiment, the lamella distance for a 1D ASG matches half the wavelength of the ultrasound center frequency. For instance, a 3MHz US (very common) has a wavelength of λ= 500µm in water, so an ASG grid of 250µm would equal λ/2. In case the ASG is 2D, in particular where the ASG has the honeycomb structure as shown in Fig 2C or similar, a cell-center-to-cell-center pitch matches half the wavelength of the ultrasound center frequency.

In one embodiment the ASG assembly is removably arranged in the detector module DM. For instance, a user operable release mechanism allows a user to mount or dismount the ASG at will in or from a housing of the module block DM. For instance, a housing of the combined module DM may include tracks with an arrest mechanism (eg, snap fit or latch mechanism) is which the AGS is slidably received and secured. Alternatively, the tracks may be formed as flanged fringe portions of the top face of the Ultrasound transducer UST and the ASG can be slipped onto the US transducer UST as and when required.

In one embodiment the user has at his or her disposal a set of different ASG grids filled with different filler materials each having different acoustic impedance. This allows to "cherry pick" the ASG from the set whose filler material has the best fitting acoustic impedance for the ultrasound task at hand. Another option is to have a single ASG assembly filled with a filler material whose acoustic impedance can be changed at will so as to match a range acoustic impedances for various tissue types. The different acoustic impedances may be achieved by a suitable treatment (eg, by heat, chemical or otherwise) to make the filler material (preferably reversibly) softer or harder to thereby bring about an acoustic impedance adjustment to different tissue types.

Rather than choosing the filler material to match a specific tissue type (fat, muscle etc) which may be appropriate in some embodiments, one may compute instead an average (possibly weighted) of acoustic impedances of different (relevant) human tissues and then chose the filler material to match the so computed average acoustic impedance. The weights represent the expected composition of a tissue mixture one wishes to image.

Reference is now made to Figure 3 which shows cross sectional side elevation views on the detector modules DM as envisaged herein in different embodiments. Although the illustrations in Fig 3 mainly relate to indirect conversion type detector assemblies XD, the following may find equal application to direct conversion type detector assemblies.

Referring first to the embodiment in Figure 3A), this shows the ASG assembly arranged on top of the detector module DM. In other words the ASG assembly in the detector module is proximal to the X-ray source with the X-ray beam XRB propagating in the direction p. The ultrasound waves propagate along directions d as shown. In the embodiment of Figure 3A, a photo diode PD detector layer 303 of the X-ray detector XD is formed on one (the distal) side of a silicone substrate (or wafer) SS whereas the transducer UTC is arranged on the other (proximal) side of the same silicon wafer SS. In other words, the substrate SS is treated on both sides when manufacturing the embodiment of Fig 3A. In the embodiment shown the X-ray detector photonic layer is a CMOS X-ray detector with integrated with Si-photon detectors, but other solutions are also envisaged. The scintillator layer 304 is coupled, distal to X-ray source XR, to the distal face of the photo-diode layer 303 from underneath.

The embodiment of Figure 3B is similar to the one shown in Figure 3A, however this time the transducer UST and parts of the detector XD are formed on separate silicon wafers SSa, SSb and these wafers are then attached back-to-back to each other as shown in Figure 3B. Preferably the ultrasound transducer UST and the X-ray detector XD are both manufactured in CMOS process on the different wafers SSa, SSb. The silicon wafers SSA, SSb are preferably thinned and then attached back-to-back. Scintillator 405 or, as the case may be a direct conversion material layer, are placed preferably distal from the X-ray source XR as this is more advantageous when mounting or joining the two substrates SSa,SSb for otherwise one must sandwich the Scintillator layer 405 or the direct conversion material layer in between. However such a sandwich solution is still envisaged in some embodiments.

Unlike the embodiment in Figure 3A where the CMOS detector layer included integrated Photon detectors PD, the embodiment in Fig 3B features a separate CMOS detector layer coupled to a large area photo-detector layer 404. However this may not be so in all embodiments and the integrated photo-detectors as in Figure 3A may be used instead in the Fig 3B embodiment. Conversely, the Fig 3A embodiment may include instead of the integrated photo-diodes, the separate, large area photo-detector layer 404 as in Fig 3B. As a further variant to the above, the ultrasound transducer UST and the X-ray detector XD can be formed on different substrates from different materials rather than both being formed on wafers of the same material. More particularly and according to one embodiment, a glass or foil-based X-ray detector layer is coupled to the back (that is, distal) side of transducer UST. Again, advantageously, the transducer is of the CMUT type, so is essentially a thin silicon wafer which is easy to handle when the X-ray detector layer is coupled thereto. The matrix of detector pixels can be made of thin-film electronics, e.g. a-Si, organic materials, amorphous oxides or LTPS (Low-temperature polycrystalline silicon).

The detector layer XD can be attached upside down to the transducer UST layer, i.e. with the scintillator or direct X-ray conversion layer distal from the X-ray source XR. Alternatively, the scintillator layer or direct X-ray conversion material layer may be mounted proximal to the X-ray source but in this case the US-transducer UST has to be mounted on top of the X-ray conversion layer or scintillator layer.

As a yet further embodiment, the transducer UST and the X-ray detector are completely autonomous devices, not sharing any physical interface but are merely arranged in a stack, not unlike hard-drive modules in a server housing. Preferably, the whole stack may be mounted in a single housing. Preferably, the US-transducer and the X-ray detector are still at least coupled to each other to ensure alignment during the concurrent US and X-ray imaging procedure. The US-transparent ASG is placed on top of the US transducer module UST. Optionally, an inter-layer is arranged between the US-transducer UST and X-ray detector XD to provide damping of the US beam to further avoid artifacts from reflections.

Any of the above described embodiments of the detector module DM can be housed in a single housing or frame.

In either one of the above embodiments, the ultrasound transducer UST is preferably of the CMUT type but other solutions such a piezo-type US transducers are also envisaged herein. Preferably, in the CMUT embodiment, the associated read-out circuitry is likewise integrated with the CMUT structure on the substrate SS or SSa, respectively. In either one of the above described embodiments of detector module DM, the AGS assembly is suitable coupled to the proximal face of the US transducer UST. This can be implemented by gluing (eg, with silicone) or the AGS is releasably coupled to the proximal face of the US transducer UST.

As can be seen in all the previously described embodiments of the detector module DM, the X-ray radiation passes through the ASG first and then through the ultrasound transducer UST.

In general, the ultrasound transducer will be arranged on top of the X-ray detector because the X-ray detector (in particular its scintillator or its direct conversion layer) is not usually transparent for ultrasound. However, if the X-ray detector assembly XD can be manufactured from a material that is ultrasound transparent it is also envisaged herein to arrange the X-ray detector on top of the ultrasound transducer, with both positioned under the ASG.

In some embodiments the footprints (surface area) of the ASG, the ultrasound transducer UST and the X-ray detector coincide in shape and surface area. However, this may not be so in all embodiments where at least the footprint of the ultrasound transducer T is smaller than the field of view or footprint of the underlying X-ray detector.

For instance, in some embodiments the ultrasound transducer UST may be arranged to run as a mere strip across the field of view of the underlying X-ray detector. As a numerical example, the transducer UST may be arranged as a strip having a width of only about 1 cm against the full width of about 10 cm of the underlying X-ray detector footprint. In case the footprint of the ultrasound transducer does not coincide with that of the X-ray detector XD, not all lamellae of the ASG need be filled with the filler material as proposed above. Only those lamellae inter-spaces are filled with filler material that are in registry with the underlying UST matrix. The remaining lamella inter-spaces may be filled with a dummy structure such as a cellulose structure or otherwise to merely serve as a stabilizer.

If one choses, for whatever reason, to fill only some of the interspaces in the grid with the filler material, one may still use a full view UST transducer but this may be operated by activating only selected parts of the transducer cell matrix that are in registry with the filled interspaces. Disturbances of the US beam can be minimized. To keep the X-ray visibility low, the rest of the X-ray detector could be covered by an unprocessed wafer of the same material stack as the US transducer. Alternatively, and less preferably, the differences in X-ray absorption can be eliminated by software gain correction of the X-ray images. The filling of ASG areas outside that US transducer footprint allows achieving homogeneous X-ray absorption throughput the complete field of view of the X-ray detector. This allows reducing image artifacts in the X-ray image.

Various methods of manufacture are envisaged for the "filled" ASG assemblies described above in Figs 1-3. In one embodiment the grid structure that is the system of lamellas is provided in an intermediate matrix structure held in the correct spatial position. This matrix structure together with the lamella is then positioned in a vacuum chamber. More particularly the matrix structure is suitably stabilized and a vacuum is built up on one side of this lamella matrix structure. The filler material is on the other side and is forced into the inter-spaces displacing the matrix material due to the suction power exerted by the vacuum. This type of vacuum assisted introduction of the filler material into the lamella inter-space can be used advantageously where the filler material is gel or other semi-fluid. However, this method of manufacture can also be used with the silicone where the silicone is provided in a liquid form is then sucked into the inter-spaces and then left to solidify. In this manner of wafer filled ASG can be produced which are then diced into the desired shape.

Using silicone RTV has the advantage that it can be prepared in a two component setup. The two-component materials are mixed into a liquid and this is then applied to interspaces between the lamella and then hardens within minutes or hours. A temporary fibrous matrix material can be used initially to hold the lamellae in place and this is then expelled or is dissolved when the filler material (such as the two component RTV silicone) is applied to the grid.

In an alternative manufacturing embodiment, the ASG structure is built up by depositing, in alternation, layers of filler material and lamella material on top of each other. For instance the filler material and the lamella material are arranged in strips and these are placed in alternation side by side or on top of each other against a worktop with a sufficiently smooth surface having formed therein a flange or stopper block. A strip of filler material (such as a silicone strip) is then placed on the worktop to rest against the flange and then one continues to deposit in alternation filler strips and lamella strips to so gradually build up the ASG assembly having a footprint that corresponds to that of the work plate. This layered structure ("ASG wafer") can then be diced into the desired shapes to arrive at the ASG assembly and is then ready for use. The filler material strips and the lamella strips are glued together in this process of alternate depositing and the surface of the work plate is such that the ASG wafer does not easily get stuck, when glue contacts the worktop. The ASG wafer can thus be released easily without incurring damage.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An anti-scatter assembly (ASG) for X-ray imaging including a grid (G) filled with a filler material (202), **characterised by** said filler material (202) having an acoustic impedance that corresponds to that of human or animal tissue.

2. Assembly of claim 1, wherein the acoustic impedance is in the range of 1.4 MRayls to 1.8 MRayls.

3. Assembly of claim 1 or 2, wherein the acoustic impedance is around 1.5 MRayls.

4. Assembly of any one of the previous claims, wherein the filler material is a gel and/or wherein the filler material includes silicone and/or wherein the filler material includes a polymer.

5. Assembly of any one of the previous claims wherein a distance between lamellae (203) of the grid (g) matches half a wavelength of an ultrasound center frequency.

6. Assembly as per any one of claims 1-4 where a pitch of the grid is smaller than a tenth of a wavelength of an ultrasound center frequency.

7. Assembly of any one of the previous claims, wherein the assembly includes a retainer component (201,204) so as to retain the filler material within the grid.

8. Assembly of any one of the previous claims, comprising a coupling structure (CO) for coupling ultrasound into a body of a human or animal patient.

9. A detector module (DM), comprising:
an ultrasound transducer (UST); and
an assembly as per any one of the previous claims coupled to said ultrasound transducer.

10. Detector module of claim 9, further comprising an X-ray detector (XD).

11. Detector module of claim 9 or 10, wherein the ultrasound transducer (UST), the ASG assembly and the X-ray detector are arranged in-line.

12. Detector module of claim 11, wherein the ASG assembly is arranged in front of said ultrasound transducer (UST) relative to an ultrasound propagation direction.

13. A combined X-ray and Ultrasound imaging system (IM) comprising a detector module as per any one of claims 9-12 or an assembly as per any one of claims 1-8.

14. An imaging system, comprising:
an X-ray imaging component (XC);
an US-imaging component (USC), and
an anti-scatter assembly as per any one of claims 1-8 for said X-ray imaging component.

15. Imaging system of claim 14, wherein the anti-scatter grid is arrangeable, during operation of the US imaging component, between an X-ray source of the X-ray component and an ultrasound transducer of said US imaging component.

## Patentansprüche

1. Streustrahlen verhindernde Anordnung (ASG) für Röntgenbildgebung, enthaltend ein mit einem Füllmaterial (202) gefülltes Raster (G), **dadurch gekennzeichnet, dass** das Füllmaterial (202) eine akustische Impedanz aufweist, die der von menschlichem oder tierischem Gewebe entspricht.

2. Anordnung nach Anspruch 1, wobei die akustische Impedanz im Bereich von 1.4 MRayls bis 1.8 MRayls liegt.

3. Anordnung nach Anspruch 1 oder 2, wobei die akustische Impedanz ungefähr 1.5 MRayls beträgt.

4. Anordnung nach einem der vorstehenden Ansprüche, wobei das Füllmaterial ein Gel ist und/oder wobei das Füllmaterial Silikon enthält und/oder wobei das Füllmaterial ein Polymer enthält.

5. Anordnung nach einem der vorstehenden Ansprüche, wobei eine Entfernung zwischen Lamellen (203) des Rasters (g) mit der halben Wellenlänge einer Ultraschall-Mittenfrequenz übereinstimmt.

6. Anordnung nach einem der Ansprüche 1-4, wobei ein Abstand des Rasters kleiner ist als ein Zehntel einer Wellenlänge einer Ultraschall-Mittenfrequenz.

7. Anordnung nach einem der vorstehenden Ansprüche, wobei die Anordnung eine Rückhaltekomponente (201, 204) so enthält, um das Füllmaterial innerhalb des Rasters zurückzuhalten.

8. Anordnung nach einem der vorstehenden Ansprüche, umfassend eine Kopplungsstruktur (CO) zum Koppeln von Ultraschall in einen Körper eines menschlichen oder tierischen Patienten.

9. Detektormodul (DM), umfassend:
einen Ultraschallwandler (UST); und
eine Anordnung nach einem der vorstehenden Ansprüche, die mit dem Ultraschallwandler gekoppelt ist.

10. Detektormodul nach Anspruch 9, weiter umfassend einen Röntgendetektor (XD).

11. Detektormodul nach Anspruch 9 oder 10, wobei der Ultraschallwandler (UST), die ASG-Anordnung und der Röntgendetektor in Linie angeordnet sind.

12. Detektormodul nach Anspruch 11, wobei die ASG-Anordnung vor dem Ultraschallwandler (UST) im Verhältnis zu einer Ultraschall-Propagationsrichtung angeordnet ist.

13. Kombiniertes Röntgen- und Ultraschall-Bildgebungssystem (IM), umfassend ein Detektormodul nach einem der Ansprüche 9-12 oder eine Anordnung nach einem der Ansprüche 1-8.

14. Bildgebungssystem, umfassend:
eine Komponente zur Röntgenbildgebung (XC);
eine Komponente zur Ultraschall-Bildgebung (USC), und
eine Streustrahlen verhindernde Anordnung nach einem der Ansprüche 1-8 für die Komponente zur Röntgenbildgebung.

15. Bildgebungssystem nach Anspruch 14, wobei das Streustrahlen verhindernde Raster während des Betriebs der Komponente zur Ultraschall-Bildgebung zwischen einer Röntgenquelle der Röntgenkomponente und einem Ultraschallwandler Komponente zur Ultraschall-Bildgebung anordenbar ist.

## Revendications

1. Ensemble anti-dispersion (ASG) pour imagerie aux rayons X comprenant une grille (G) remplie d'un matériau de charge (202), **caractérisé en ce que** ledit matériau de charge (202) a une impédance acoustique qui correspond à celle d'un tissu humain ou animal.

2. Ensemble selon la revendication 1, dans lequel l'impédance acoustique se situe dans la plage de 1,4 Mrayl à 1,8 Mrayl.

3. Ensemble selon la revendication 1 ou 2, dans lequel l'impédance acoustique est d'environ 1,5 Mrayl.

4. Ensemble selon l'une quelconque des revendications précédentes, dans lequel le matériau de charge est un gel et/ou dans lequel le matériau de charge comprend une silicone et/ou dans lequel le matériau de charge comprend un polymère.

5. Ensemble selon l'une quelconque des revendications précédentes, dans lequel une distance entre les lamelles (203) de la grille (g) correspond à la moitié d'une longueur d'onde d'une fréquence centrale des ultrasons.

6. Ensemble selon l'une quelconque des revendications 1 à 4, dans lequel un pas de la grille est plus petit qu'un dixième d'une longueur d'onde d'une fréquence centrale des ultrasons.

7. Ensemble selon l'une quelconque des revendications précédentes, dans lequel l'ensemble comprend un composant de retenue (201, 204) pour retenir le matériau de charge dans la grille.

8. Ensemble selon l'une quelconque des revendications précédentes, comprenant une structure de couplage (CO) pour coupler des ultrasons dans le corps d'un patient humain ou animal.

9. Module détecteur (DM) comprenant :
un transducteur ultrasonique (UST) ; et
un ensemble selon l'une quelconque des revendications précédentes couplé audit transducteurs ultrasonique.

10. Module détecteur selon la revendication 9, comprenant en outre un détecteur de rayons X (XD).

11. Module détecteur selon la revendication 9 ou 10, dans lequel le transducteur ultrasonique (UST), l'ensemble ASG et le détecteur de rayons X sont agencés en ligne.

12. Module détecteur selon la revendication 11, dans lequel l'ensemble ASG est agencé devant ledit transducteur ultrasonique (UST) par rapport à une direction de propagation des ultrasons.

13. Système combiné d'imagerie à rayons X et à ultrasons (IM) comprenant un module détecteur selon l'une quelconque des revendications 9 à 12 ou un ensemble selon l'une quelconque des revendications 1 à 8.

14. Système d'imagerie comprenant :
un composant d'imagerie à rayons X (XC) ;
un composant d'imagerie à ultrasons (USC) et
un ensemble anti-dispersion selon l'une quelconque des revendications 1 à 8 pour ledit composant d'imagerie à rayons X.

15. Système d'imagerie selon la revendication 14, dans lequel la grille anti-dispersion peut être agencée, au cours du fonctionnement du composant d'imagerie à ultrasons, entre une source de rayons X du composant à rayons X et un transducteur ultrasonique dudit composant d'imagerie à ultrasons.
